# EUROPEAN PATENT APPLICATION

(11) **EP 4 410 770 A1**
(43) Date of publication of application: **07.08.2024**
(21) Application number: 22876216.7
(22) Date of filing: 27.09.2022
(51) Int. Cl.: C07C 51/487, C07C 51/493, C07C 53/16, C07C 231/02, C07C 233/05, C07H 21/04, C12N 15/11

(54) **PRODUCTION METHOD FOR PURIFIED DICHLOROACETIC ACID**

(30) Priority: 28.09.2021 JP 2021158346
(71) Applicant: Sumitomo Chemical Company, Limited, Tokyo 103-6020 (JP)
(72) Inventor: KANO, Toshifumi, Osaka-shi, Osaka 5550021 (JP); TANAKA, Yuki, Oita-shi, Oita 8700106 (JP); KAWAI, Hayato, Oita-shi, Oita 8700106 (JP)
(74) Representative: Marks & Clerk LLP
(86) International application number: PCT/JP2022/035920
(87) International publication number: WO 2023/054350

(57) **Abstract**

The present invention provides a production method for purified dichloroacetic acid and an efficient production method for a nucleic acid molecule using the same. Specifically, the present invention provides a production method for purified dichloroacetic acid having a molar ratio of formaldehyde to dichloroacetic acid of 81 × 10⁻⁵ or less and a molar ratio of dichloroacetic anhydride to dichloroacetic acid of 20 × 10⁻⁵ or less, the method including: bringing dichloroacetic acid having both or one of formaldehyde content and dichloroacetic anhydride content exceeding the ratio(s), into contact with at least one compound having a boiling point lower than that of dichloroacetic acid selected from the group consisting of aliphatic alcohols, aliphatic amines, and water, and, in the coexistence of an aprotic inert solvent having a boiling point lower than that of dichloroacetic acid, distilling off a fraction containing the solvent from the resulting mixed liquid, and a production method for a nucleic acid molecule using the purified dichloroacetic acid as a deprotecting agent.

## Description

### TECHNICAL FIELD

This application claims priority to and the benefit of Japanese Patent Application No. 2021-158346 filed on September 28, 2021, the entire contents of which are incorporated herein by reference.

The present invention relates to a production method for purified dichloroacetic acid, an analysis method thereof, and a method for synthesizing a nucleic acid molecule by an amidite method using purified dichloroacetic acid.

### BACKGROUND ART

In recent years, the application of nucleic acid molecules to the medical field has attracted increasing interest. Examples of the nucleic acid molecules include antisense nucleic acids, aptamers, ribozymes, and nucleic acids which induce RNA interference (RNAi) such as siRNAs, which are referred to as nucleic acid therapeutics.

A nucleic acid molecule can be synthesized by a solid-phase synthesis method, a nucleic acid molecule synthesized by elongating a nucleic acid on a solid support is cleaved from the solid support, and for the nucleic acid molecule including a ribose, a protecting group for a hydroxyl group at the 2'-position of the ribose is removed by deprotection to produce target nucleic acid molecule. In the solid-phase synthesis method, a phosphoramidite (hereinafter, referred to as an "amidite") of a nucleoside is known to be used as a raw material, and a protecting group for a hydroxyl group at the 5'-position is known to be deprotected with the use of a dichloroacetic acid solution, but the yield of a nucleic acid molecule synthesized with the use of a dichloroacetic acid solution may not necessarily been satisfactory, thereby resulting an inefficient synthesis (Patent Document 1).

### PRIOR ART DOCUMENTS

### PATENT DOCUMENT

Patent Document 1: WO 99/43694 A

### SUMMARY OF THE INVENTION

### PROBLEMS TO BE SOLVED BY THE INVENTION

An object of the present invention is to provide an efficient production method for a nucleic acid molecule.

### MEANS FOR SOLVING THE PROBLEMS

As a result of intensive studies to achieve the above object, the present inventors provide an efficient production method for a nucleic acid molecule, using, in the synthesis of a nucleic acid molecule, a purified dichloroacetic acid solution containing formaldehyde and dichloroacetic anhydride that are equal to or less than certain amounts, or dichloroacetic acid after improving the quality of the dichloroacetic acid, and an analysis method for the dichloroacetic acid.

The present invention encompasses, but is not limited to, the following aspects.
1. A production method for purified dichloroacetic acid having a molar ratio of formaldehyde to dichloroacetic acid of 81 × 10⁻⁵ or less and a molar ratio of dichloroacetic anhydride to dichloroacetic acid of 20 × 10~ ⁵ or less, the method including:
   bringing dichloroacetic acid having both or one of formaldehyde content and dichloroacetic anhydride content exceeding the ratio(s),
   into contact with at least one compound having a boiling point lower than that of dichloroacetic acid selected from the group consisting of aliphatic alcohols, aliphatic amines, and water, and, in the coexistence of an aprotic inert solvent having a boiling point lower than that of dichloroacetic acid, distilling off a fraction containing the solvent from the resulting mixed liquid (hereinafter, the production method is referred to as the purification method of the present invention).
2. The production method according to the item 1, wherein the aprotic inert solvent is an aprotic inert solvent having a boiling point of 181°C or lower.
3. The production method according to the item 1 or 2, wherein the aprotic inert solvent is dichloromethane, acetonitrile, or an aromatic organic solvent.
4. The production method according to the item 3, wherein the aromatic organic solvent is toluene.
5. The production method according to any one of the items 1 to 4, wherein the aliphatic alcohol is a C1-C6 aliphatic alcohol.
6. The production method according to any one of the items 1 to 5, wherein the purified dichloroacetic acid has a molar ratio of formaldehyde to dichloroacetic acid of 41 × 10⁻⁵ or less.
7. The production method according to any one of the items 1 to 6, wherein the purified dichloroacetic acid has a molar ratio of dichloroacetic anhydride to dichloroacetic acid of 10 × 10⁻⁵ or less.
8. The production method according to any one of the items 1 to 7, wherein the purified dichloroacetic acid has a molar ratio of formaldehyde to dichloroacetic acid of 81 × 10⁻⁶ or less.
9. The production method according to any one of the items 1 to 8, wherein the purified dichloroacetic acid has a molar ratio of dichloroacetic anhydride to dichloroacetic acid of 50 × 10⁻⁶ or less.
10. A production method for a nucleic acid molecule by an amidite method, the method including the steps of: preparing purified dichloroacetic acid having a molar ratio of formaldehyde to dichloroacetic acid of 81 × 10⁻⁵ or less and a molar ratio of dichloroacetic anhydride to dichloroacetic acid of 20 × 10⁻⁵ or less; and reacting the purified dichloroacetic acid with a nucleic acid molecule wherein a hydroxyl group at the 5'-terminal is protected to remove a protecting group for the hydroxyl group.
11. The production method according to the item 10, wherein the preparation step is the method according to any one of the items 1 to 9.
12. The production method according to the item 10 or 11, wherein the nucleic acid molecule wherein a hydroxyl group at the 5'-terminal is protected is a nucleic acid molecule represented by formula (1): 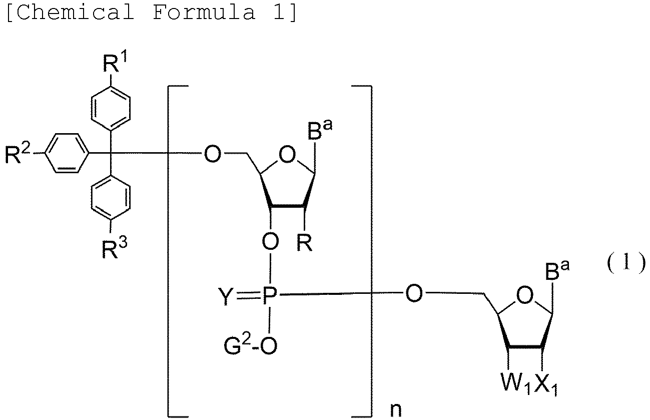 (in the formula,
   G² represents a protecting group for a hydroxyl group,
   B^{a} is the same or different and each independently represents a nucleobase optionally protected with a protecting group,
   R¹, R² and R³ are the same or different and each independently represent a hydrogen atom or an alkoxy group,
   R is the same or different and each independently represents a protected hydroxyl group, a hydrogen atom, a fluorine atom, a methoxy group, a 2-methoxyethyl group, or an OQ' group,
   Q' is the same or different and each independently represents a methylene group bonded to a carbon atom at a 4'-position of a ribose, an ethylene group bonded to a carbon atom at a 4'-position of a ribose, or an ethylidene group bonded to a carbon atom at a 4'-position of a ribose;
   Y is the same or different and each independently represents an oxygen atom or a sulfur atom,
   n represents any integer of 1 to 200,
   W₁ represents an OZ group, and X₁ represents an R group, or
   W₁ represents an OV group, and X₁ represents an OZ group,
   V represents a protecting group for a hydroxyl group, and
   Z is a group having a structure consisting of a solid support and a linking group, and
   when n is an integer of 2 or more, a non-nucleotide linker may be incorporated between respective nucleotides in the nucleic acid molecule represented by the formula (1)), and
   the deprotected nucleic acid molecule is a nucleic acid molecule represented by formula (2): 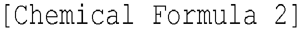 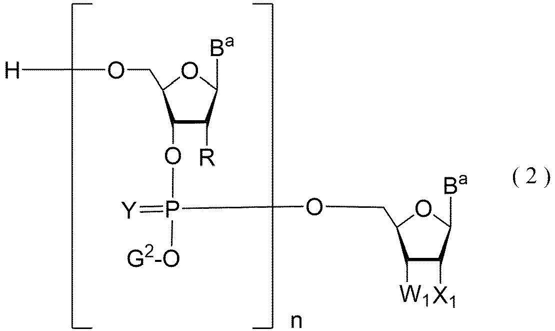 (in the formula,
      G², B^{a}, R, Y, X₁, W₁, and n are as described above, and
      a non-nucleotide linker may be incorporated between nucleotides as defined in the formula (1)).
13. A production method for a nucleic acid molecule represented by formula (2'), the method including: the step according to the item 12; and further a step of removing a group represented by Z from the nucleic acid molecule represented by the formula (2), produced in the step according to the item 12; and a step of removing protecting groups for a hydroxyl group and a nucleobase, 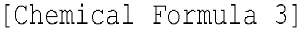 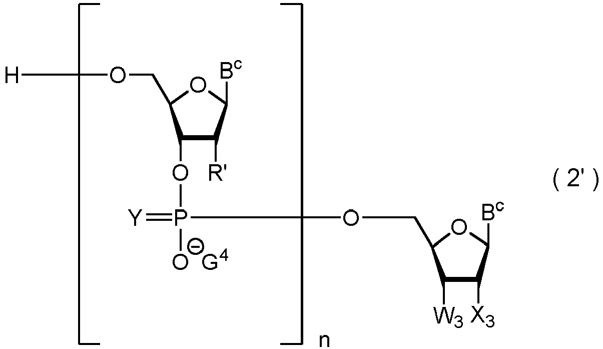 (in the formula,
   Y and n are as described above,
   B^{c} is the same or different and each independently represents a nucleobase,
   G⁴ is the same or different and each independently represents a hydrogen ion, an alkali metal ion, an ammonium ion, an alkylammonium ion, or a hydroxyalkylammonium ion,
   R' is the same or different and each independently represents a hydroxyl group, a hydrogen atom, a fluorine atom, a methoxy group, a 2-methoxyethyl group, or an OQ' group,
   Q' is as described above, and
   X₃ and W₃ each independently represent a hydroxyl group, or
   X₃ represents an R' group, and W₃ represents a hydroxyl group, and,
   a non-nucleotide linker may be incorporated between nucleotides as defined in the formula (1)).
14. The production method according to any one of the items 10 to 13, wherein the nucleic acid molecule is a nucleic acid molecule containing ribonucleic acid (RNA).
15. The production method according to the item 14, wherein the nucleic acid molecule is a ribonucleic acid (RNA), and a protecting group for a hydroxyl group at a 2'-position of a ribose of the ribonucleic acid is a protecting group represented by formula (6),
   Formula (6): 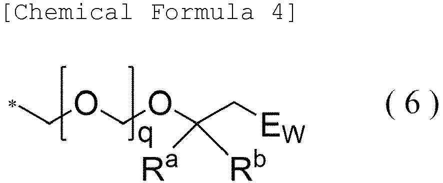 (in the formula,
   q represents any integer of 0 to 5,
   R^{a} and R^{b} are the same or different and each independently represent a methyl group, an ethyl group, or a hydrogen atom,
   mark * represents a site bonded to an oxygen atom derived from the hydroxyl group at the 2'-position of the ribose, and
   E_{w} represents an electron-withdrawing group).
16. The production method according to the item 15, wherein one of R^{a} and R^{b} is a methyl group, the other is a hydrogen atom, and E_{w} is a cyano group.
17. The production method according to any one of the items 10 to 16, wherein the nucleic acid molecule is an oligomer with a chain length of 40 or more.
18. The production method according to any one of the items 10 to 16, wherein the nucleic acid molecule is an oligomer with a chain length of 50 or more.
19. The production method according to any one of the items 10 to 16, wherein the nucleic acid molecule is an oligomer with a chain length of 60 or more.
20. The production method according to any one of the items 10 to 16, wherein the nucleic acid molecule is an oligomer with a chain length of 80 or more.
21. The production method according to any one of the items 10 to 16, wherein the nucleic acid molecule is an oligomer with a chain length of 100 or more.
22. An analysis method for dichloroacetic anhydride contained in a dichloroacetic acid reagent, the method including: reacting a dichloroacetic acid reagent containing dichloroacetic anhydride with an arylalkylamine to convert the dichloroacetic anhydride to a corresponding 2,2-dichloro-N-arylalkylacetamide; and analyzing the resulting amide by high performance liquid chromatography.
23. The analysis method according to the item 22, wherein
   the arylalkylamine is a compound of formula (I): 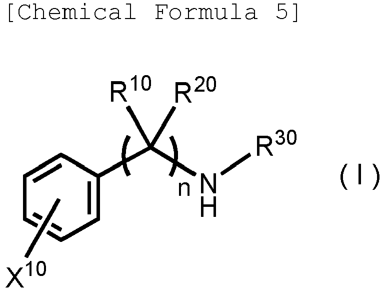 (in the formula,
   R¹⁰, R²⁰, and R³⁰ are the same or different and each independently represent a hydrogen atom or an alkyl group,
   X¹⁰ represents a hydrogen atom, an alkyl group, or an alkoxy group, and
   n is an integer of 1 to 5), and
   the 2,2-dichloro-N-arylalkylacetamide is an amide compound of formula (II): 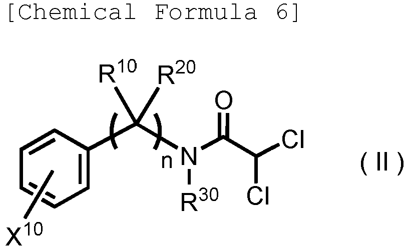 (in the formula, R¹⁰, R²⁰, R³⁰, X¹⁰ and n are as described above).
24. The analysis method according to the item 23, wherein R¹⁰, R²⁰, and R³⁰ are the same or different and each independently represent a hydrogen atom or a C1-C6 alkyl group, and X¹⁰ is a hydrogen atom, a C1-C6 alkyl group, or a C1-C6 alkoxy group.
25. The analysis method according to any one of the items 22 to 24, wherein 0.01 to 3.0 mol of the arylalkylamine is used per 1 mol of dichloroacetic acid.
26. The analysis method according to any one of the items 22 to 24, wherein 0.05 to 2.0 mol of the arylalkylamine is used per 1 mol of dichloroacetic acid.
27. The analysis method according to any one of the items 22 to 24, wherein 0.08 to 1.1 mol of the arylalkylamine is used per 1 mol of dichloroacetic acid.
28. The analysis method according to any one of the items 22 to 27, wherein the arylalkylamine is benzylamine, and the amide compound is 2,2-dichloro-N-benzylacetamide.
29. The analysis method according to any one of the items 22 to 28, wherein the reaction between the dichloroacetic acid reagent containing dichloroacetic anhydride and the arylalkylamine is performed using acetonitrile as a solvent.

### EFFECT OF THE INVENTION

The present invention provides a production method for purified dichloroacetic acid and an efficient production method for a nucleic acid molecule using the same. The yield of the nucleic acid molecule to be produced can be expected to be improved by the production method of the present invention.

### BRIEF DESCRIPTION OF THE DRAWINGS

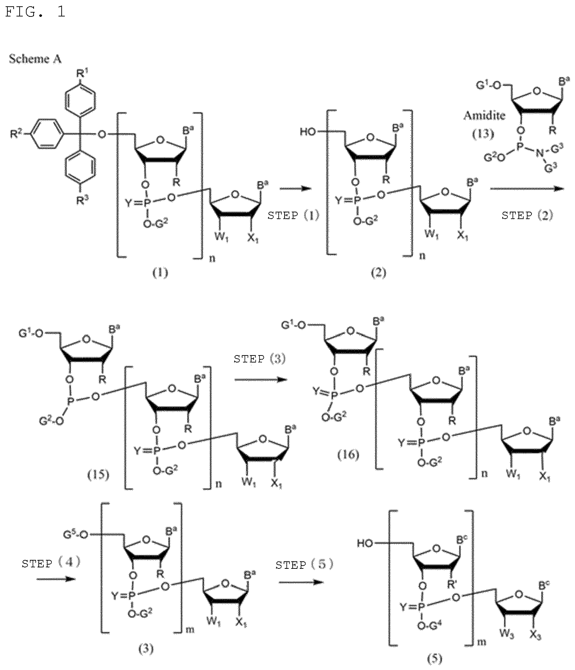

Fig. 1 is a Scheme A showing a typical example of producing a nucleic acid molecule represented by formula (5) from a nucleic acid molecule represented by the formula (1). In the figure, any group can be used as G¹ without any particular limitation as long as the group can function as a protecting group for a hydroxyl group to be removed by dichloroacetic acid, and known protecting groups used in amidite compounds can be widely used. In addition, G³ is the same or different and each independently represents an alkyl group, or two G³s may be bonded to each other to form a cyclic structure. Preferably, G³ is the same or different and each independently represents an alkyl group, for example, a methyl group, an ethyl group, a propyl group, or an isopropyl group, and both G³s are more preferably isopropyl groups. The other symbols are as described above.

### MODE FOR CARRYING OUT THE INVENTION

A production method for purified dichloroacetic acid having a molar ratio of formaldehyde to dichloroacetic acid of 81 × 10⁻⁵ or less and a molar ratio of dichloroacetic anhydride to dichloroacetic acid of 20 × 10⁻⁵ or less will be described.

Examples of the dichloroacetic acid having both or one of formaldehyde content and dichloroacetic anhydride content exceeding the ratio(s) used in the production method for purified dichloroacetic acid include commercially available dichloroacetic acid reagents or those produced by a known method. The amounts of formaldehyde and dichloroacetic anhydride contained in these dichloroacetic acid reagents and the like can be analyzed and used.

Examples of the dichloroacetic acid to be subjected to purification of the present invention include 1) dichloroacetic acid having a molar ratio of formaldehyde to dichloroacetic acid exceeding 81 × 10⁻⁵ and a molar ratio of dichloroacetic anhydride to dichloroacetic acid exceeding 20 × 10⁻⁵, 2) dichloroacetic acid having a molar ratio of formaldehyde to dichloroacetic acid of 81 × 10⁻⁵ or less and a molar ratio of dichloroacetic anhydride to dichloroacetic acid exceeding 20 × 10⁻⁵, and 3) dichloroacetic acid having a molar ratio of formaldehyde to dichloroacetic acid exceeding 81 × 10⁻⁵ and a molar ratio of dichloroacetic anhydride to dichloroacetic acid of 20 × 10⁻⁵ or less. According to the purification method of the present invention, these dichloroacetic acids can be purified to reduce the formaldehyde content and dichloroacetic anhydride content to the ranges of the desired ratios of the purification method of the present invention.

In the purification method of the present invention, at least one compound having a boiling point lower than that of dichloroacetic acid selected from the group consisting of aliphatic alcohols, aliphatic amines, and water may be added to dichloroacetic acid having both or one of formaldehyde content and dichloroacetic anhydride content exceeding the ratio(s), and then an aprotic inert solvent having a boiling point lower than that of dichloroacetic acid may be added, or at least one compound selected from the group consisting of aliphatic alcohols, aliphatic amines, and water, having a boiling point lower than that of dichloroacetic acid may be added after an aprotic inert solvent having a boiling point lower than that of dichloroacetic acid is added in advance to a dichloroacetic acid reagent to be produced.

Examples of the aliphatic alcohol and aliphatic amine having a boiling point lower than that of dichloroacetic acid include C1-C6 aliphatic alcohols and C1-C6 aliphatic amine compounds. Examples of the C1-C6 aliphatic alcohol include methanol, ethanol, n-propanol, i-propanol, n-butanol, i-butanol, sec-butanol, t-butanol, n-pentanol, and n-hexanol. Examples of the aliphatic amine having a boiling point lower than that of dichloroacetic acid include C1-C6 aliphatic amine compounds. Specific examples thereof include methylamine, ethylamine, propylamine, butylamine, pentylamine, and hexylamine.

The amount of the aliphatic alcohol, the aliphatic amine, and water or a mixture thereof used is not particularly limited as long as it is an amount effective for reducing dichloroacetic anhydride to the desired range. The amount of dichloroacetic anhydride can be analyzed by an analysis method for dichloroacetic anhydride contained in dichloroacetic acid reagent described below to determine the effective amount.

Examples of the aprotic inert solvent having a boiling point lower than that of dichloroacetic acid include aprotic inert solvents having a boiling point of 181°C or lower, and specific examples thereof include aromatic organic solvents (for example, toluene, xylene, monochlorobenzene, and o-dichlorobenzene), acetonitrile, and dichloromethane. The amount of such a solvent used is not particularly limited, but is typically, for example, about 0.5 to 20 times by weight of dichloroacetic acid.

The bath temperature in distillation off is usually 20°C to 120°C, and may be under atmospheric pressure or under reduced pressure. The distillation off of the lowboiling point component mainly including the solvent does not need to distill off all of the solvent, and may be partial distillation off. The distillation off is usually performed in such a manner that the amount of the solvent and the like distilled off is in the range of 50% to 75% of the amount of the solvent added and used. In this way, a purified dichloroacetic acid solution of the desired amounts of dichloroacetic anhydride and formaldehyde is obtained.

In nucleic acid molecule synthesis by an amidite method, the purified dichloroacetic acid preferably has a molar ratio of dichloroacetic anhydride to dichloroacetic acid of 10 × 10⁻⁵ or less and more preferably has a molar ratio of dichloroacetic anhydride to dichloroacetic acid of 50 × 10⁻⁶ or less.

The purified dichloroacetic acid preferably has a molar ratio of formaldehyde to dichloroacetic acid of 41 × 10⁻⁵ or less and more preferably has a molar ratio of formaldehyde to dichloroacetic acid of 81 × 10⁻⁶ or less.

The analysis method for dichloroacetic anhydride contained in a dichloroacetic acid reagent will be described.

The analysis method is usually performed by reacting a predetermined amount of sample of a dichloroacetic acid reagent containing dichloroacetic anhydride with an arylalkylamine in an inert solvent to convert the dichloroacetic anhydride to the corresponding 2,2-dichloro-N-arylalkylacetamide, and analyzing the produced amide by high performance liquid chromatography (HPLC).

Examples of the arylalkylamine include the compounds of the formula (I), and examples of the 2,2-dichloro-N-arylalkylacetamide produced include the compounds of the formula (II).

In the formulas (I) and (II), the alkyl group in R¹⁰, R²⁰, R³⁰ or X¹⁰ is preferably a C1-C6 alkyl group, and more preferably a methyl group. The alkoxy group in X¹⁰ is preferably a C1-C6 alkoxy group, and more preferably a methoxy group. R¹⁰, R²⁰, R³⁰ or X¹⁰ is preferably a hydrogen atom, and n = 1. The arylalkylamine is preferably benzylamine.

The amount of the arylalkylamine used is typically 0.01 to 3.0 mol, preferably 0.05 to 2.0 mol, and more preferably 0.08 to 1.1 mol, based on 1 mol of dichloroacetic acid.

The reaction of a dichloroacetic acid reagent containing dichloroacetic anhydride with an arylalkylamine is typically carried out in acetonitrile as a solvent.

Analysis of 2,2-dichloro-N-arylalkylacetamide by HPLC is usually performed with the use of an ODS column. As mobile phases, analysis is performed with, for example, a gradient using a formic acid aqueous solution as a mobile phase A and using acetonitrile as a mobile phase B. The UV detection wavelength is typically 254 nm.

Next, a production method for a nucleic acid molecule by an amidite method, the method including the steps of: preparing purified dichloroacetic acid having a molar ratio of formaldehyde to dichloroacetic acid of 81 × 10⁻⁵ or less and a molar ratio of dichloroacetic anhydride to dichloroacetic acid of 20 × 10⁻⁵ or less; and reacting the purified dichloroacetic acid with a nucleic acid molecule wherein a hydroxyl group at the 5'-terminal is protected to remove the protecting group will be described.

The purified dichloroacetic acid used is prepared by the steps as described above. When the purified dichloroacetic acid is obtained by a method other than the method described above, it may be selected from among them.

Examples of the protecting group for the hydroxyl group at the 5'-position of the nucleic acid molecule include a group represented by G⁵ below. Examples of the nucleic acid compound wherein the hydroxyl group at the 5'-position is protected include the nucleic acid compound of the formula (1). Examples of the nucleic acid compound produced by reacting the purified dichloroacetic acid solution include the nucleic acid compound of the formula (2) .

Specific examples of the compounds that are the same or different and each independently represent a methylene group bonded to a carbon atom at the 4'-position of a ribose, an ethylene group bonded to a carbon atom at the 4'-position of a ribose, or an ethylidene group bonded to a carbon atom at the 4'-position of a ribose, represented by Q', in the formulas (1) and (2) include structures represented by LNA-1, LNA-2, or LNA-3 of the following formula (7). 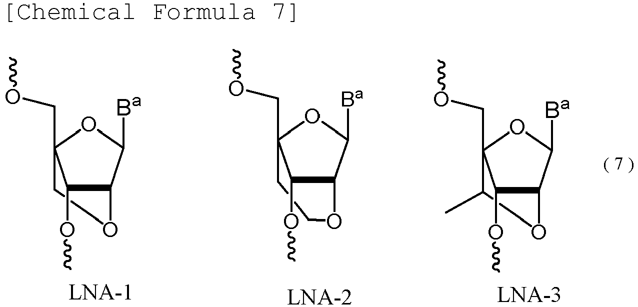 (In the formula, B^{a} represents an optionally protected nucleobase).

More specific examples of a group having a structure including a solid support and a linking group connecting the solid support and an oxygen atom of a hydroxyl group at the 2'-position or 3'-position of a ribose at the 3'-terminal of the nucleic acid molecule, represented by Z, include a structure represented by the following formula (8) .

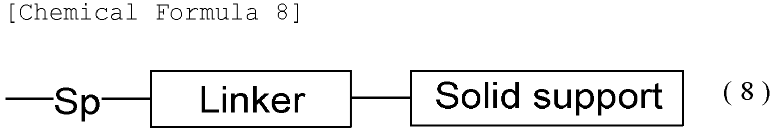

In the formula (8), Sp represents a spacer.

Examples of the spacer (Sp) include spacers having a structural formula represented by the following formula (9) .

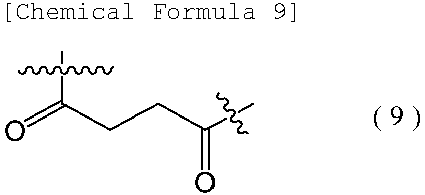

The Linker may have, for example, a structure represented by the following formula (10), or may have a structure represented by the formula (10) without any hexamethylene amino group moiety and with an aminopropyl group bonded to Si. Alternatively, the Linker may have a structure represented by the following formula (11). 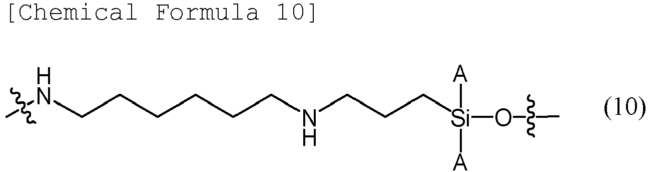 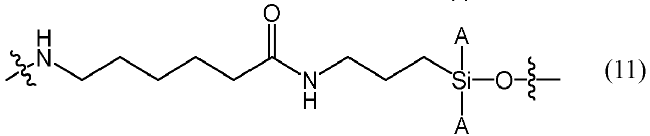 (In the formula,
A may be any of a hydroxyl group, an alkoxy group, and an alkyl group. Examples of the alkoxy group include a methoxy group and an ethoxy group. Examples of the alkyl group include a methyl group, an ethyl group, an isopropyl group, and an n-propyl group. Si indicates that Si is bonded to oxygen of a hydroxyl group on the support surface.)

Examples of the Solid support include an inorganic porous support and an organic resin support. Examples of the inorganic porous support include Controlled Pore Glass (CPG). Examples of the organic resin support include a support made of polystyrene.

Examples of nucleosides (ribose and deoxyribose) contained in the nucleic acid molecule for use in the present invention include DNA, RNA, 2'-O-MOE (2'-O-methoxyethyl), 2'-O-Me, 2'-F RNA, and the aforementioned LNAs, but the nucleoside is not limited thereto.

The method for synthesizing a nucleic acid molecule by a solid-phase synthesis method, including a deprotection step with the purified dichloroacetic acid solution, typically includes the following steps:
(1) a step of deprotecting a hydroxyl group at the 5'-position of a nucleoside wherein a hydroxyl group is protected and which is bonded to a solid support via a linker;
(2) a step of subjecting the hydroxyl group at the 5'-position produced in the previous step, to a coupling reaction with a phosphoramidite compound to obtain a phosphite triester compound;
(3) a step of oxidizing the phosphite triester produced in the previous step to be converted into a phosphate triester, thereby producing an elongated nucleic acid molecule, or an optional step of converting the phosphite triester into a thiophosphate triester;
(4) a step of synthesizing a nucleic acid molecule on the solid support by repeating, any number of times, a series of reaction cycles composed of the steps (1) to (3), that is, the step of deprotecting the hydroxyl group at the 5'-position of the produced nucleic acid molecule, the step of coupling the hydroxyl group at the 5'-position with the amidite compound, and the step of oxidizing the produced phosphite triester; and
(5) a step of subjecting the nucleic acid molecule on the solid support produced in the step (4), to a step of cleaving and deprotecting the nucleic acid molecule, and releasing the nucleic acid molecule from the solid support to produce a nucleic acid molecule with a protecting group removed therefrom.

The method for synthesizing a nucleic acid molecule may, however, include, following the step (2) or (3), a step of capping the hydroxyl group at the 5'-position where the coupling reaction with the phosphoramidite compound fails to proceed, or the capping step may be added between any steps of the series of reaction cycles constituting the step (4).

The step (5) is more specifically performed by subjecting the nucleic acid molecule on the solid support, produced in the step (4), to the reactions of the following steps (5-1) and (5-2) in this order, and then subjecting the nucleic acid molecule to the reaction of the step (5-3). In this regard, the reaction of the step (5-1) may be performed optionally, and the reaction of the step (5-2) may be performed with the use of the method described in JP 4705716 B. As a result, a nucleic acid molecule with a protecting group removed from a nucleic acid molecule released from the solid support or a nucleic acid molecule with a protected hydroxyl group at the 5'-terminal can be produced.
(5-1) a reaction of deprotecting a protecting group for the hydroxyl group at the 5'-terminal of the nucleic acid molecule;
(5-2) a reaction of cleaving and releasing the nucleic acid molecule from the solid support; and
(5-3) a reaction of deprotecting a protecting group for a hydroxyl group at the 2'-position of a ribose constituting the nucleic acid molecule or the 3'-position of the 3'-terminal thereof.

The scheme of the steps (1) to (5) is shown in Fig. 1. The deprotection reaction in the step (1) or step (4) shown in Fig. 1 is performed with the use of the purified dichloroacetic acid solution. The definitions of the substituents in the chemical formulas in the Scheme A are as defined above.

The nucleic acid compound of the formula (1) can be further elongated by any chain length with the use of a nucleotide-type or non-nucleotide-type linker by an amidite method, and used for the production of the nucleic acid compound represented by the formula (3). The nucleic acid molecule represented by the formula (5) can be also obtained by cleaving only the nucleic acid compound from the nucleic acid compound bonded to the solid support, of the formula (3), and then further deprotecting the nucleic acid compound. Hereinafter, the substituents in each formula will be described in more detail.

The nucleobase optionally protected by the protecting group, represented by B^{a}, and the nucleobase represented by B^{c} are not particularly limited. Examples of the nucleobases include adenine, cytosine, guanine, uracil, thymine, 5-methylcytosine, pseudouracil, and 1-methylpseudouracil. In addition, the nucleobases may be substituted with a substituent. Examples of such a substituent include a halogen atom such as a fluoro group, a chloro group, a bromo group, or an iodo group, an acyl group such as an acetyl group, an alkyl group such as a methyl group or an ethyl group, an arylalkyl group such as a benzyl group, an alkoxy group such as a methoxy group, an alkoxyalkyl group such as a methoxyethyl group, a cyanoalkyl group such as a cyanoethyl group, a hydroxy group, a hydroxyalkyl group, an acyloxymethyl group, an amino group, a monoalkylamino group, a dialkylamino group, a carboxy group, a cyano group, and a nitro group, and combinations of two or more of these substituents.

The protecting group for the nucleobase, represented by B^{a}, and optionally protected with a protecting group, is not particularly limited, known protecting groups used in nucleic acid chemistry can be used, and examples of such protecting groups include a benzoyl group, a 4-methoxybenzoyl group, a 4-methylbenzoyl group, an acetyl group, a propionyl group, a butyryl group, an isobutyryl group, a phenylacetyl group, a phenoxyacetyl group, a 4-tert-butylphenoxyacetyl group, a 4-isopropylphenoxyacetyl group, and a (dimethylamino)methylene group, and combinations of two or more of these groups.

B^{a} more specifically represents a group represented by any of the following: 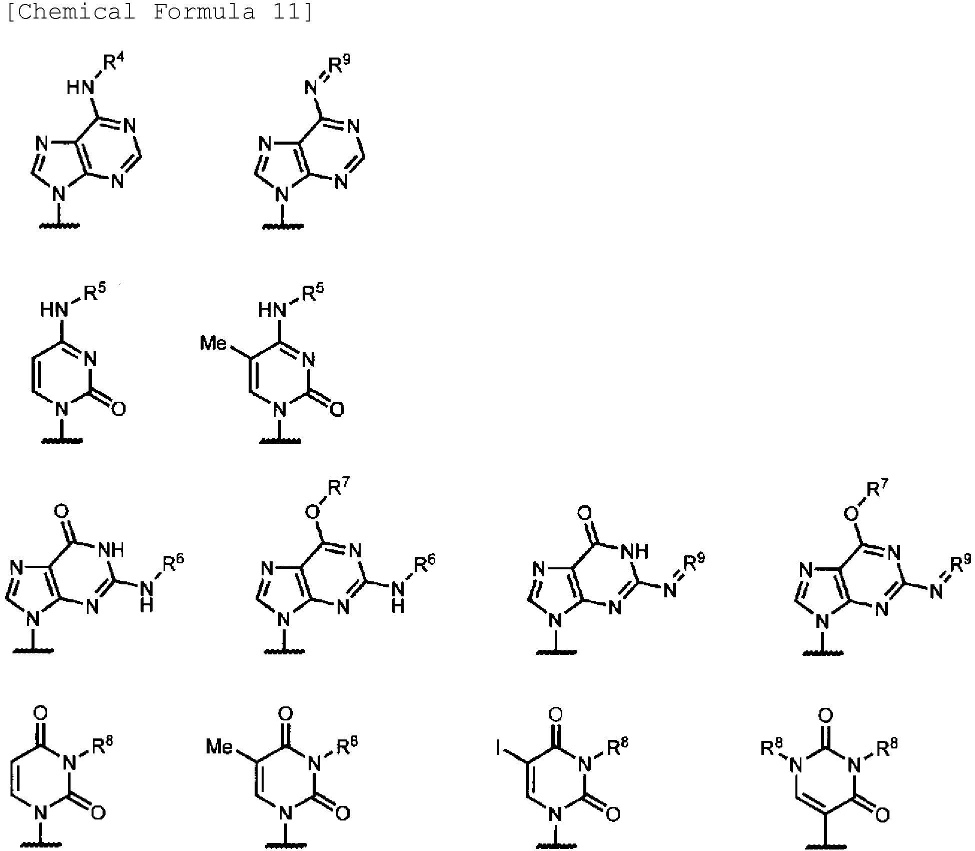 (in the formula described above,
R⁴ represents a hydrogen atom, a methyl group, a phenoxyacetyl group, a 4-tert-butylphenoxyacetyl group, a 4-isopropylphenoxyacetyl group, a phenylacetyl group, an acetyl group, or a benzoyl group,
R⁵ represents a hydrogen atom, an acetyl group, an isobutyryl group, or a benzoyl group,
R⁶ represents a hydrogen atom, a phenoxyacetyl group, a 4-tert-butylphenoxyacetyl group, a 4-isopropylphenoxyacetyl group, a phenylacetyl group, an acetyl group, or an isobutyryl group,
R⁷ represents a 2-cyanoethyl group,
R⁸ represents a hydrogen atom, a methyl group, a benzoyl group, a 4-methoxybenzoyl group, or a 4-methylbenzoyl group, and
R⁹ represents a dimethylaminomethylene group).

More specific examples of B^{c} include groups obtained by removing the protecting groups from the above-described specific examples of B^{a}.

G¹ and G⁵ are preferably the following groups. 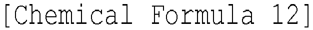 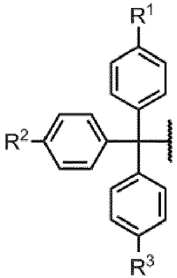 (in the formula,
R¹, R² and R³ are the same or different and each independently represent a hydrogen atom or an alkoxy group) .

Preferably, one of R¹, R² and R³ is a hydrogen atom, whereas the remaining two thereof are alkoxy groups which are the same or different (preferably the same), and the alkoxy groups are particularly preferably methoxy groups. More preferably, G⁵ is a 4,4'-dimethoxytrityl group (DMTr group) .

Any group can be used as G² without any particular limitation as long as the group can function as a protecting group for a hydroxyl group, and known protecting groups used in amidite compounds can be widely used. Examples of G² include an alkyl group, an alkenyl group, an alkynyl group, a cycloalkyl group, a haloalkyl group, an aryl group, a heteroaryl group, an arylalkyl group, a cycloalkenyl group, a cycloalkylalkyl group, a cyclylalkyl group, a hydroxyalkyl group, an aminoalkyl group, an alkoxyalkyl group, a heterocyclylalkenyl group, a heterocyclylalkyl group, a heteroarylalkyl group, a silyl group, a silyloxyalkyl group, a mono-, di-, or trialkylsilyl group, and a mono-, di-, or trialkylsilyloxyalkyl group, and these groups are optionally substituted with one or more electron-withdrawing groups.

G² is preferably an alkyl group substituted with an electron-withdrawing group. Examples of the electron-withdrawing group include a cyano group, a nitro group, an alkylsulfonyl group, a halogen atom, an arylsulfonyl group, a trihalomethyl group, and a trialkylamino group, and a cyano group is preferred.

Particularly preferred as G² are the following group.

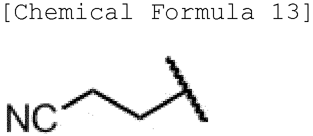

The alkyl groups in the definitions of R¹, R², R³, and G² may be linear or branched, and are preferably alkyl groups having 1 to 12 carbon atoms, and more preferably alkyl groups having 1 to 6 carbon atoms. Specific examples of the alkyl groups include a methyl group, an ethyl group, a n-propyl group, an isopropyl group, a n-butyl group, an isobutyl group, a tert-butyl group, a n-pentyl group, an isopentyl group, and a hexyl group. The alkyl group moiety constituting the alkoxy group in the definition of the substituent has the same definition as the definition of the alkyl group herein.

In addition, in the method of the present invention, the amidite compound can be used in a free state or a salt state. Examples of the salt of the amidite compound include, but not be particularly limited to, a base addition salt or an acid addition salt. Specific examples of the base addition salt include salts with inorganic bases, such as a sodium salt, a magnesium salt, a potassium salt, a calcium salt, and an aluminum salt; salts with organic bases, such as a methylamine, an ethylamine, and an ethanolamine; salts with basic amino acids, such as a lysine, an ornithine, and an arginine; and ammonium salts. Specific examples of the acid addition salt include mineral acids such as hydrochloric acid, hydrobromic acid, hydroiodic acid, sulfuric acid, nitric acid, and phosphoric acid; organic acids such as formic acid, acetic acid, propionic acid, oxalic acid, malonic acid, malic acid, tartaric acid, fumaric acid, succinic acid, lactic acid, maleic acid, citric acid, methanesulfonic acid, trifluoromethanesulfonic acid, and ethanesulfonic acid; and acid addition salts with acidic amino acids such as aspartic acid and glutamic acid. The amidite compound also encompasses forms such as salts, hydrates, solvates, and crystal polymorphs.

R preferably represents a protected hydroxyl group. The protecting group in the case of R representing a protected hydroxyl group or the protecting group for the hydroxyl group represented by V may be any group that can be used in the amidite method, and for example, the groups described in WO 2013/027843 A and WO 2019/208571 A can be used in addition to a 2'-tert-butyldimethylsilyl (TBS) group, a 2'-bis(2-acetoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group, a 2'-para-toluylsulfonylethoxymethyl (TEM) group, and a 2'-EMM group (WO 2006/022323 A). V is preferably a 2'-tert-butyldimethylsilyl (TBS) group. In addition, when the nucleic acid molecule includes therein a ribose, for example, when the nucleic acid molecule produced by the method of the present invention is ribonucleic acid (RNA), as a protecting group for the hydroxyl group at the 2'-position of the ribose, the protecting group represented by the formula (6) is exemplified as a preferred protecting group. More preferably, a protecting group represented by formula (12) having a cyano group as an electron-withdrawing group represented by E_{w} is exemplified. 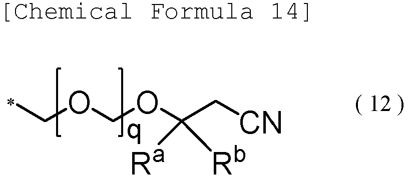 (in the formula,
q, R^{a}, and R^{b} have the same meaning as defined in the formula (6)).

More preferably, a group where q is 1 and R^{a} and R^{b} are simultaneously hydrogen atoms, and a group where q is 1, one of R^{a} and R^{b} is a methyl group, and the other is a hydrogen atom are exemplified for the group represented by the formula (12).

The protecting group represented by the formula (12) can be synthesized, for example, in accordance with the description of WO 2013/027843 A and WO 2019/208571 A, and an amidite compound having such a protecting group can be used for the production of a nucleic acid compound.

The amidite compound of formula (13) listed in the Scheme A of Fig. 1 is used for the elongation reaction of the nucleic acid.

Examples of the non-nucleotide linker include a linker including an amino acid backbone (for example, a linker including the amino acid backbone described in JP 5157168 B or JP 5554881 B). Specifically, non-limiting examples of the non-nucleotide linker include a linker represented by formula (A14-1) or (A14-2) or (A14-3) (described in, for example, WO 2019/074110 A). Besides these linkers, examples of the non-nucleotide linker include the linkers described in WO 2012/005368 A, WO 2018/182008 A, or WO 2019/074110 A. 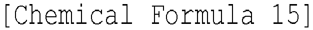 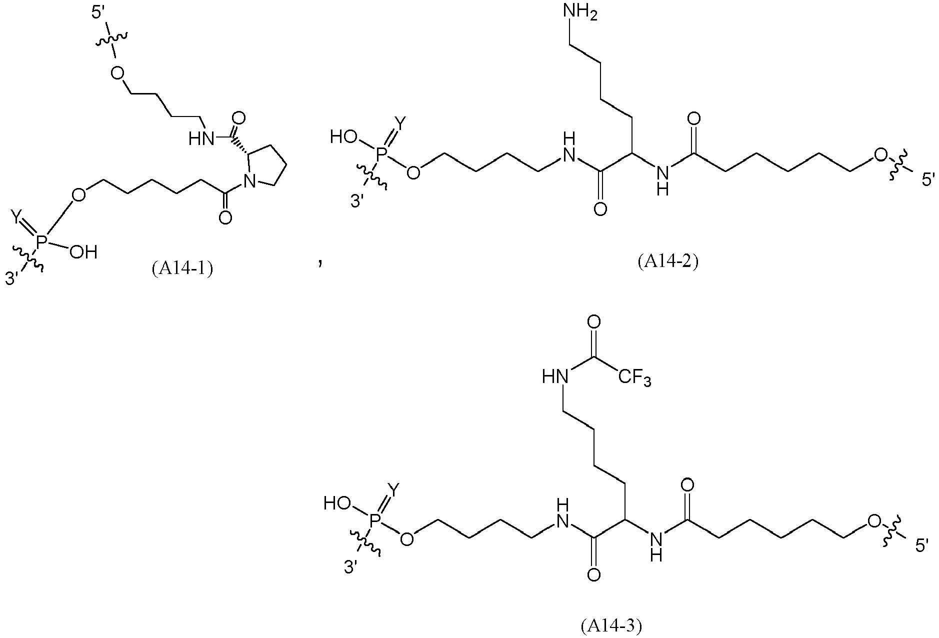 (in the formula, Y is as described above).

Nucleotides and amidites where the R group in the formula (13) and the R' group in the formula (5) are substituents other than a hydroxyl group can also be produced from nucleosides synthesized by known methods as described in JP 3745226 B and the like, and WO 2001/053528 A or JP 2014-221817 A, and the known methods cited therein, and furthermore, with the use of those available as commercial products, can be produced in accordance with the methods described in examples as described later or by the method to which appropriate changes are made.

G⁴ represents a hydrogen atom, an alkali metal ion, an ammonium ion, an alkylammonium ion, or a hydroxyalkylammonium ion. Examples of the alkali metal ion include a sodium ion and a lithium ion. In addition, as the alkylammonium ion, specific examples of the alkyl group include a methyl group, an ethyl group, a n-propyl group, an isopropyl group, a n-butyl group, an isobutyl group, a tert-butyl group, a n-pentyl group, an isopentyl group, and a hexyl group, and more specific examples thereof include a diethylammonium ion, a triethylammonium ion, a tetrabutylammonium ion, a hexylammonium ion, and a dibutylammonium ion. Moreover, as the hydroxyalkylammonium ion, specific examples of the hydrokyalkyl moiety include hydroxymethyl, hydroxyethyl, hydroxy-n-propyl, hydroxyisopropyl, hydroxy-n-butyl, and trishydroxymethyl, and more specific examples of the hydroxyalkylammonium ion include a trishydroxymethylammonium ion. G⁴ preferably represents a hydrogen atom.

G⁵ represents a hydrogen atom or a protecting group for the hydroxyl group, and when G⁵ represents a protecting group, G¹ also represents the same protecting group. G⁵ is a hydrogen atom in the case of being deprotected, but the nucleotide compound in that case is also subjected to the series of steps for the nucleic acid elongation reaction.

Y is preferably an oxygen atom.

As for W₁ and X₁, preferably, W₁ represents an OZ group, and X₁ represents an R group.

As for W₂ and X₂, preferably, W₂ represents a hydroxyl group, and X₂ represents an R group.

W₃ and X₃ preferably each independently represent a hydroxyl group.

R' is preferably a hydroxyl group.

For the synthesis of the nucleic acid compound by the amidite method from the steps (1) to (5), a nucleic acid elongation reaction can be performed in accordance with a generally known method (for example, the method described in JP 5157168 B or JP 5554881 B), except for the deprotecting step according to the present invention in the step (1) or step (5) in the scheme of Fig. 1. Each step will be described below.

### (Nucleic Acid Elongation Reaction)

In this specification, the "nucleic acid elongation reaction" means a reaction of sequentially binding nucleotides via phosphodiester bonds to elongate an oligonucleotide. The nucleic acid elongation reaction can be performed in accordance with the procedure of a common phosphoramidite method. The nucleic acid elongation reaction may be performed with the use of an automatic nucleic acid synthesizer or the like in which a phosphoramidite method is employed.

The chain length of the nucleic acid molecule may be, for example, 20 mer or more (that is, n ≥ 19), 40 mer or more (that is, n ≥ 39), 50 mer or more (that is, n ≥ 49), 60 mer or more (that is, n ≥ 59), 80 mer or more (that is, n ≥ 79), 100 mer or more (that is, n ≥ 99), 2 to 200 mer (that is, 1 ≤ n ≤ 199), 10 to 150 mer (that is, 9 ≤ n ≤ 149), or 15 to 110 mer (that is, 14 ≤ n ≤ 109).

The deprotection step of the step (1) is a step of deprotecting the protecting group for the 5'-hydroxyl group at the terminal of the oligonucleotide chain supported on the solid support. As a common protecting group, a 4,4'-dimethoxytrityl group (DMTr group), a 4-monomethoxytrityl group, or a 4,4',4"-trimethoxytrityl group is used. The deprotection can be performed with the use of an acid. Examples of the acid for the deprotection include trifluoroacetic acid, dichloroacetic acid, trifluoromethanesulfonic acid, trichloroacetic acid, methanesulfonic acid, hydrochloric acid, acetic acid, and p-toluenesulfonic acid.

The coupling step of the step (2) is a reaction in which a nucleoside phosphoramidite represented by the following formula (13) shown in the Scheme A of Fig. 1 is bound to the 5'-hydroxyl group at the terminal of the oligonucleotide chain deprotected by the deprotection step. Examples of the phosphoramidite used for the nucleic acid elongation include the formula (13), the uridine EMM amidite described in Example 2 of JP 5554881 B, the cytidine EMM amidite described in Example 3, the adenosine EMM amidite described in Example 4, and the guanosine EMM amidite described in Example 5, the uridine PMM amidite, the cytidine PMM amidite, the adenosine PMM amidite, and the guanosine PMM amidite described in WO 2019/208571 A. In addition, other examples of the phosphoramidite that can be used include 2'-OMe, 2'-F, a 2'-O-tert-butyldimethylsilyl group, a 2'-O-methoxyethyl group, a 2'-bis(2-acetoxy)methyl (ACE) group, a 2'-(triisopropylsilyloxy)methyl (TOM) group, a 2'-(2-cyanoethoxy)ethyl (CEE) group, a 2'-(2-cyanoethoxy)methyl (CEM) group, a 2'-para-toluylsulfonylethoxymethyl (TEM) group, 2'-H, and a 2'-fluoro-2'-deoxy-β-D-arabinofuranosyl group. As the nucleoside phosphoramidite, a 5'-hydroxyl group protected with a protecting group (e.g., DMTr group) is used. The coupling step can be performed with the use of an activating agent or a coupling agent that activates the nucleoside phosphoramidite. Examples of the activating agent or coupling agent include 5-benzylthio-1H-tetrazole (BTT) (also referred to as 5-benzylmercapto-1H-tetrazole), 1H-tetrazole, 4,5-dicyanoimidazole (DCI), 5-ethylthio-1H-tetrazole (ETT), N-methylbenzimidazolium triflate (N-MeBIT), benzimidazolium triflate (BIT), N-phenylimidazolium triflate (N-PhIMT), imidazolium triflate (IMT), 5-nitrobenzimidazolium triflate (NBT), 1-hydroxybenzotriazole (HOBT), or 5-(bis-3,5-trifluoromethylphenyl)-1H-tetrazole.

The nucleoside phosphoramidite represented by the formula (13) (hereinafter, referred to as an amidite) shown in the Scheme A of Fig. 1 is as follows.

A compound represented by the formula: 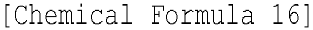 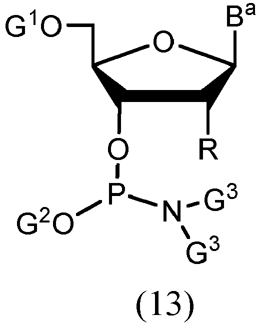 (in the formula,
G¹, G², G³, B^{a}, and R are as described above) .

After the coupling step, the unreacted 5'-hydroxyl group may be capped as appropriate. The capping can be performed with the use of a known capping solution such as an acetic anhydride-tetrahydrofuran solution or a phenoxyacetic anhydride/N-methylimidazole solution.

The oxidation step of the step (3) is a step of converting the phosphite group formed in the coupling step into a phosphate group or a thiophosphate group. This step is a reaction of converting trivalent phosphorus into pentavalent phosphorus with the use of an oxidizing agent, and can be performed by reacting the oxidizing agent with the oligonucleic acid derivative supported on the solid support.

In the case of converting the phosphite group into a phosphate group, for example, iodine can be used as the "oxidizing agent". The oxidizing agent can be prepared so as to have a concentration of 0.005 to 2 M, and used. Water can be used as an oxygen source for the oxidation, and pyridine, N-methylimidazole (NMI), N-methylmorpholine, triethylamine, or the like can be used as a base for allowing the reaction to proceed. In addition, the solvent is not particularly limited as long as the solvent is not involved in the reaction, and examples thereof include acetonitrile, tetrahydrofuran (THF), or mixed solvents in arbitrary proportions thereof. For example, iodine/water/pyridine/acetonitrile, or iodine/water/pyridine, or iodine/water/pyridine/NMI, or iodine/water/pyridine/THF can be used. The reaction temperature is preferably 5°C to 50°C. The reaction time is usually appropriately 1 minute to 30 minutes. The amount of the reagent used is preferably 1 to 100 mol, and more preferably 1 to 10 mol based on 1 mol of the compound supported on the solid support.

In the case of converting the phosphite triester group to a thiophosphate triester group, for example, sulfur, 3H-1,2-benzodithiol-3-one-1,1-dioxide (Beaucage reagent), 3-amino-1,2,4-dithiazole-5-thione (ADTT), 5-phenyl-3H-1,2,4-dithiazol-3-one (POS), [(N,N-dimethylaminomethylidene)amino]-3H-1,2,4-dithiazoline-3-thione (DDTT), and phenyl acetyl disulfide (PADS) can be used as the "oxidizing agent". The oxidizing agent can be diluted with an appropriate solvent so as to have a concentration of 0.001 to 2 M, and used. The solvent for use in the reaction is not particularly limited as long as the solvent is not involved in the reaction, and examples thereof include dichloromethane, acetonitrile, pyridine, or mixed solvents in arbitrary proportions thereof. The oxidation step may be performed after the capping operation, or conversely, the capping operation may be performed after the oxidation step, and this order is not limited.

In the step (5-1), the protecting group for the hydroxyl group at the 5'-position of the nucleotide introduced at the end of the elongation may be used for column purification with the protecting group for the hydroxyl group at the 5'-position as a tag after cleaving from the solid support and deprotecting the protecting group as described later, or the protecting group for the hydroxyl group at the 5'-position may be deprotected after column purification.

In the step (5-2), in the step of deprotecting the phosphate protecting group, after completing the synthesis of the nucleic acid that has a desired sequence, an amine compound is allowed to act for deprotecting the protecting group for the phosphate moiety. Examples of the amine compound include diethylamine described in JP 4705716 B.

The cleavage of the nucleic acid molecule elongated to a desired chain length on the solid support from the solid support in the step (5-2) is usually performed with the use of concentrated aqueous ammonia as a cleavage agent.

Further, for example, the oligonucleotide chain is cleavage from the solid support with the use of ammonia, an amine compound, or the like, and then collected. Examples of the amine compound include methylamine, ethylamine, isopropylamine, ethylenediamine, or diethylamine.

In the step (5-3), the protecting group for the hydroxyl group at the 2'-position or 3'-position of a ribose of the nucleic acid compound (4) cleaved from the solid support in the step (5-2) can be removed in accordance with the method described in WO 2006/022323 A), WO 2013/027843 A, or WO 2019/208571 A, and a deprotected nucleic acid molecule (5) can be obtained.

Examples of the nucleic acid molecule that can be produced with the use of the production method of the present invention include, but are not limited to, nucleic acid molecules with a nucleoside included therein being RNA, DNA, RNA having 2'-O-MOE, 2'-O-Me, or 2'-F, and LNA. Examples of various nucleosides are provided, for example, as described in Xiulong, Shen et al., Nucleic Acids Research, 2018, Vol. 46, No. 46, 1584-1600, and Daniel O'Reilly et al., Nucleic Acids Research, 2019, Vol. 47, No. 2, 546-558. Preferably, the nucleic acid molecule produced by the method of the present invention is RNA.

Typical examples of the nucleic acid molecule that can be used in the production method of the present invention are, in addition to the examples described in examples, shown in the following examples, but are not limited thereto.

Hereinafter, in the descriptions of sequences, U represents uridine, C represents cytidine, A represents adenosine, and G represents guanosine.

Examples thereof include the nucleic acid molecules having the following sequences (A) and (B), described in WO 2019/060442 A.
Sequence (A): 5'-AUGGAAUmACUCUUGGUUmACdTdT-3' (Antisense) (SEQ ID NO: 1) 21 mer
Sequence (B): 5'-GUmAACmCmAAGAGUmAUmUmCmCmAUmdTdT-3' (Sense) (SEQ ID NO: 2) 21 mer

In the sequences (A) and (B), Um represents 2'-O-methyluridine, Cm represents 2'-O-methylcytidine, and dT represents thymidine.

Examples thereof include the nucleic acid molecules (see page 553) described in Daniel O'Reilly et al., Nucleic Acids Research, 2019, Vol. 47, No. 2, 546-558. Typical examples include nucleic acid molecules having the following sequence (C).
Sequence (C): 5'-AGAGCCAGCCUUCUUAUUGUUUUAGAGCUAUGCUGU-3' (SEQ ID NO: 3) 36 mer

Examples thereof include the nucleic acid molecules described in JP 4965745 B. Typical examples include nucleic acid molecules having the following sequence (D).
Sequence (D): 5'-CCAUGAGAAGUAUGACAACAGCC-P-GGCUGUUGUCAUACUUCUCAUGGUU-3' (SEQ ID NOs: 4 and 5) 49 mer

In the sequence (D), "P" is represented by a partial structure delimited by a wavy line in the following formula (A5) .

It is to be noted that the description of SEQ ID NO: 4 in the sequence listing indicates the base sequence of the following sequence (D1) from the 5'-terminal of the sequence (D) to before "P" thereof, and the description of SEQ ID NO: 5 therein indicates the base sequence of the following sequence (D2) from after "P" of the sequence (D) to the 3'-terminal thereof.
Sequence (D1): 5'-CCAUGAGAAGUAUGACAACAGCC-3' (SEQ ID NO: 4) 23 mer
Sequence (D2): 5'-GGCUGUUGUCAUACUUCUCAUGGUU-3' (SEQ ID NO: 5) 25 mer

Examples thereof include the nucleic acid molecules having the following sequence (E), described in Nucleic Acids Research, 2019, Vol. 47, No. 2: 547.
Sequence (E): 5'-ACAGCAUAGCAAGUUAAAAUAAGGCUAGUCCGUUAUCAACUUGAAAAAGUGGCACCGAG UCGGUGCU-3' (SEQ ID NO: 6) 67 mer

Examples thereof include the nucleic acid molecules having the following sequence (F), described in JP 2015-523856 A, page 173.
Sequence (F): 5'-GUUUUCCCUUUUCAAAGAAAUCUCCUGGGCACCUAUCUUCUUAGGUGCCCUCCCUUGUU UAAACCUGACCAGUUAACCGGCUGGUUAGGUUUUU-3' (SEQ ID NO: 7) 94 mer

Examples thereof include the nucleic acid molecules described in JP 2017-537626 A. Typical examples include nucleic acid molecules having the following sequences (G), (H), (K), and (J).
Sequence (G): 5'-AGUCCUCAUCUCCCUCAAGCGUUUUAGAGCUAGUAAUAGCAAGUUAAAAUAAGGCUAGU CCGUUAUCAACUUGAAAAAGUGGCACCGAGUCGGUGCUUUU-3' (SEQ ID NO: 8) 100 mer
Sequence (H): 5'-GCAGAUGUAGUGUUUCCACAGUUUAAGAGCUAUGCUGGAAACAGCAUAGCAAGUUUAAA UAAGGCUAGUCCGUUAUCAACUUGAAAAAGUGGCACCGAGUCGGUGCUUUUUUU-3' (SEQ ID NO: 9) 113 mer
Sequence (K): 5'-dAdGdTdCdCdTdCdAdTdCdTdCdCdCdTdCdAdAdGdCGUUUAAGAGCUAUGCUGGU AACAGCAUAGCAAGUUUAAAUAAGGCUAGUCCGUUAUCAACUUGAAAAAGUGGCACCGA GUCGGUGCUUUUUUU-3' (SEQ ID NO: 10) 113 mer

In the sequence (K), dT represents thymidine, dC represents 2'-deoxycytidine, dA represents 2'-deoxyadenosine, and dG represents 2'-deoxyguanosine.
Sequence (J): 5'-AmsGmsUmsCCUCAUCUCCCUCAAGCGUUUAAGAGCUAUGCUGGUAACAGCAUAGCAAG UUUAAAUAAGGCUAGUCCGUUAUCAACUUGAAAAAGUGGCACCGAGUCGGUGCUUUUms UmsUmsU-3' (SEQ ID NO: 11) 113 mer

In the sequence (J), Um represents 2'-O-methyluridine, Am represents 2'-O-methyladenosine, Gm represents 2'-O-methylguanosine, and s represents phosphorothioate modification.

### EXAMPLES

Hereinafter, the present invention will be described in more detail with reference to examples, but the present invention is not limited to these examples.

### <Measurement Method>

First, various measurement methods used in the following tests are shown below.

The oligonucleotide purity was measured with the use of HPLC.

The HPLC measurement conditions are shown in Table 1 below.

### (Measurement Method 1: Measurement of Oligonucleotide Purity)

### [Table 1]

**Table 1**

| | |
|---|---|
| Column | DNAPacTM PA200 4 × 250 mm |
| Flow rate | 1.0 mL/min |
| Detection Wavelength | 260 nm |
| Mobile Phase A | 25 mM Tris-HCl buffer (pH = 8.0), 10% CH₃CN, 6M Urea Water |
| Mobile Phase B | 500 mM NaClO₄, 25 mM Tris-HCl buffer (pH = 8.0), 10% CH₃CN, 6M Urea Water |
| Gradient Condition | B conc. 20% (0 min) - 60% (60 min) - 90% (60.01 min) - 90% (65 min) - 20% (65.01 min) - 20% (80 min) |
| Column Temperature | 80°C |

### (Measurement Method 2: Measurement of Oligonucleotide Yield)

The OD₂₆₀ of a crude product was measured. The OD₂₆₀ represents the absorbance at UV 260 nm per 10 mm optical path length in a 1 mL solution (pH = 7.5). Generally, RNA is known to be 1 OD = 40 pg, and thus, the yield was calculated based on the measurement value of OD₂₆₀.

### (Measurement Method 3: Measurement of Formaldehyde Concentration)

As a method of measuring the formaldehyde concentration in the dichloroacetic acid solution, there is high-performance liquid chromatography method. In the high-performance liquid chromatography method, formaldehyde and acetylacetone are reacted, the amount of 3,5-diacetyl-1,4-dihydrolutidine obtained is measured, and the concentration of formaldehyde is calculated.

The HPLC measurement conditions are shown in Table 2 below.

### [Table 2]

**Table 2**

| | |
|---|---|
| Column | InertSustain C18 (4.6 mm × 150 mm, 3 µm) |
| Flow rate | 1.0 mL/min |
| Mobile Phase A | Phosphoric acid/water mixed solution (1:1000) |
| Mobile Phase B | Acetonitrile |
| Isocratic Condition | B cone (%) 25% (0-10 min) |
| Detection Wavelength | 413 nm |
| Column Temperature | 40°C |

### (Measurement Method 4: Measurement of Dichloroacetic Anhydride Concentration)

As a method of measuring the dichloroacetic anhydride concentration in the dichloroacetic acid solution, there is high-performance liquid chromatography method. In the high-performance liquid chromatography method, dichloroacetic anhydride and benzylamine are reacted, the amount of N-benzyl-2,2-dichloroacetamide obtained is measured, and the concentration of dichloroacetic anhydride is calculated. The HPLC measurement conditions are shown in Table 3 below.

### [Table 3]

**Table 3**

| | |
|---|---|
| Column | MC-Pack ODS-A (4.6 mm × 150 mm, 5 µm) |
| Column Temperature | 30°C |
| Flow rate | 1.0 mL/min |
| Mobile Phase A | 0.1% Formic acid aqueous solution |
| Mobile Phase B | Acetonitrile |
| Detection Wavelength | 254 nm |
| Gradient Condition | B conc. 30% (0 min) - 50% (30 min) - 90% (30.1 min) - 90% (35 min) - 30% (35.1 min) - 30% (45 min) |

### <Preparation of Dichloroacetic Acid Solution>

Dichloroacetic acid solutions used in Examples 4 to 6 below were prepared by treating commercially available dichloroacetic acid having a molar ratio of dichloroacetic acid and formaldehyde (formaldehyde mol/dichloroacetic acid mol) of 16 × 10⁻⁴ and a molar ratio of dichloroacetic anhydride and dichloroacetic acid of 25 × 10⁻⁵ as described in Examples 1 to 3, respectively, and adding toluene to the resulting dichloroacetic acid. The molar ratio of dichloroacetic acid and formaldehyde (formaldehyde mol/dichloroacetic acid mol) and the molar ratio of dichloroacetic anhydride and dichloroacetic acid in the prepared toluene solution of dichloroacetic acid are as shown in Table 5. The number of moles of dichloroacetic acid was calculated based on the amount of a reagent weighed. It was confirmed by GC-MS that chloral was not contained. Also, in other commercially available dichloroacetic acid, the molar ratios of formaldehyde and dichloroacetic anhydride are shown in the following table for reference.

### [Table 4]

**Table 4**

| Molar ratio of each impurity to dichloroacetic acid | Company A | Company B | Company C |
|---|---|---|---|
| Formaldehyde (mol) / dichloroacetic acid (mol) | 79 × 10⁻⁴ | 19 × 10⁻⁴ | 28 × 10⁻⁴ |
| Dichloroacetic anhydride (mol) / dichloroacetic acid (mol) | 33 × 10⁻⁵ | 15 × 10⁻⁴ | 12 × 10⁻⁴ |

### Example 1

To 92.4 g of the commercially available dichloroacetic acid, were added 1.17 g of methanol and 51.2 g of toluene, the mixture was stirred at 40°C for 2 hours, and then decompressed to 33 hPa with an evaporator to distill off a low-boiling fraction. Toluene was added to the resulting dichloroacetic acid to prepare a 3 v/v% dichloroacetic acid solution. This solution was used in Example 4. Formaldehyde (mol)/dichloroacetic acid (mol) and dichloroacetic anhydride (mol)/dichloroacetic acid (mol) are shown in Table 5.

### Comparative Example 1

To 93.1 g of the commercially available dichloroacetic acid, were added 1.17 g of methanol and 1687 g of toluene to prepare a 3 v/v% dichloroacetic acid solution, and the solution was allowed to stand at room temperature. This solution was used in Comparative Example 4.

### Example 2

To 92.7 g of the commercially available dichloroacetic acid, were added 1.17 g of water and 50.6 g of toluene, the mixture was stirred at 40°C for 2 hours, and then decompressed to 33 hPa with an evaporator to distill off a low-boiling fraction. Toluene was added to the resulting dichloroacetic acid to prepare a 3 v/v% dichloroacetic acid solution. This solution was used in Example 5.

### Comparative Example 2

To 92.5 g of the commercially available dichloroacetic acid, were added 1.17 g of water and 1687 g of toluene to prepare a 3 v/v% dichloroacetic acid solution, and the solution was allowed to stand at room temperature. This solution was used in Comparative Example 5.

### Example 3

To 92.0 g of the commercially available dichloroacetic acid, were added 1.17 g of propylamine and 52.3 g of toluene, the mixture was stirred at 40°C for 2 hours, and then decompressed to 33 hPa with an evaporator to distill off a low-boiling fraction. Toluene was added to the resulting dichloroacetic acid to prepare a 3 v/v% dichloroacetic acid solution. This solution was used in Example 6.

### Comparative Example 3

217.0 g of the commercially available dichloroacetic acid was heated in an oil bath at 120°C and distilled at 40 hPa until the fraction exceeded 50%, and toluene was added to the obtained flask residue to prepare a 3 v/v% dichloroacetic acid solution. This solution was used in Comparative Example 6.

### <Solid-Phase Synthesis of Oligonucleotide 50 mer>

Sequence (I): 5'-AAGGCUAGUCCGUUAUCAACUUGAAAAAGUGGCACCGAGUCGGUGCUUUU-3' (SEQ ID NO: 12)

In the sequence (I), "A" is represented by a partial structure delimited by a wavy line in the following formula (A1). "C" is represented by a partial structure delimited by a wavy line in the following formula (A2). "G" is represented by a partial structure delimited by a wavy line in the following formula (A3). U is represented by a partial structure delimited by a wavy line in the following formula (A4). Further, "U" at the 3'-terminal is represented by a partial structure delimited by a wavy line in the following formula (A8). In addition, in the sequence (I), "A" at the 5'-terminal is represented by a partial structure delimited by a wavy line in the following formula (A7).

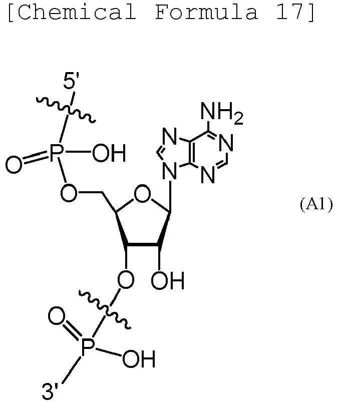

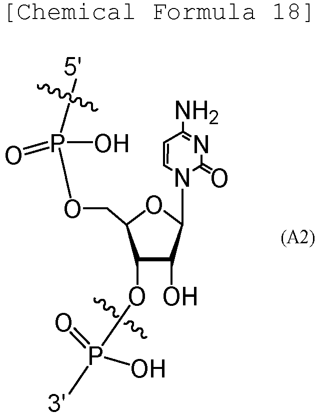

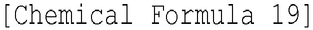

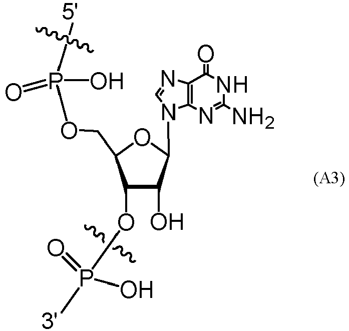

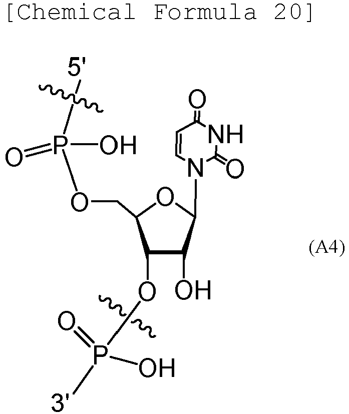

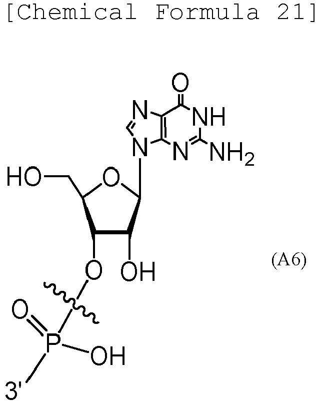

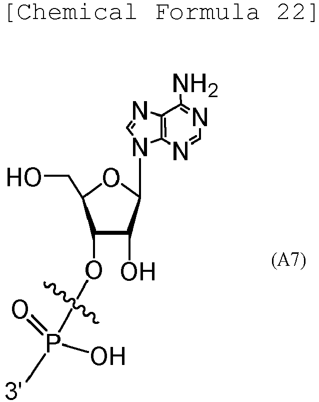

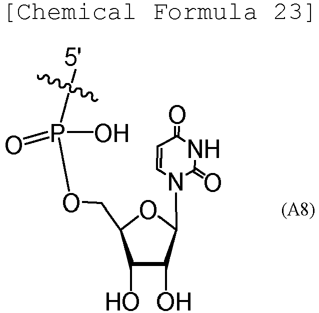

For the synthesis, the uridine PMM amidite, the cytidine PMM amidite, the adenosine PMM amidite, and the guanosine PMM amidite described in WO 2019/208571 A were used.

The uridine derivative described in the following examples and comparative examples means a compound represented by the following structural formula. The circle illustrated in the following structural formula is intended to schematically show a CPG.

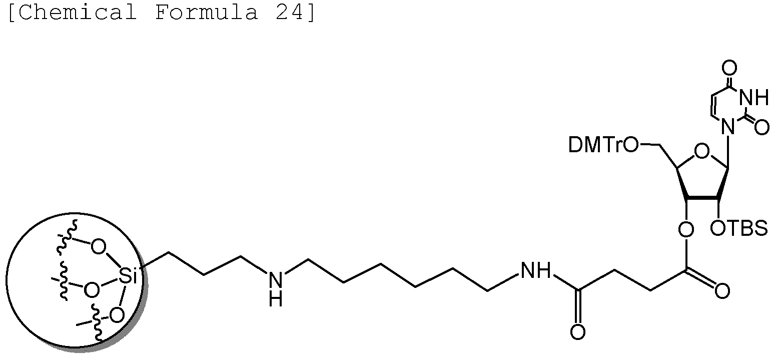

### (Example 4)

With the use of Controlled Pore Glass (CPG) with 1.0 pmol of the uridine derivative supported thereon and the PMM amidite, the nucleic acid molecule was automatically synthesized from the 3' side toward the 5' side by NTS M-4MX-E (manufactured by NIHON TECHNO SERVICE CO., LTD.). In the automatic synthesis procedure, first, a 3% dichloroacetic acid toluene solution prepared in Example 1 was sent to the CPG to deprotect the trityl protecting group at the 5'-position. Subsequently, various amidites and 5-benzylmercapto-1H-tetrazole as a coupling agent were sent to the CPG to allow a coupling reaction to proceed for the hydroxyl group at the 5'-position. Subsequently, an oxidizing solution containing 50 mM iodine was sent to convert the phosphite group into a phosphate group. Subsequently, with the use of a 0.1 M phenoxyacetic anhydride acetonitrile solution and a 10% N-methylimidazole/10% 2,6-lutidine acetonitrile solution as capping solutions, reactive sites where no coupling proceeded were capped. Further, after repeating these steps 49 times in total, the protecting group (DMTr group) of the 5'-terminal base was deprotected with a 3% dichloroacetic acid toluene solution to synthesize a nucleic acid oligonucleotide on the CPG support. Thereafter, 1.5 mL of 28% aqueous ammonia and 0.5 mL of ethanol were allowed to flow into the CPG support with 1.0 pmol of the oligonucleotide supported thereon, and the mixture was kept at 40°C for 4 hours to release the nucleic acid molecule from the solid support, and then concentrated to remove the solvent. Next, the free oligonucleotide was dissolved in 1.5 mL of dimethyl sulfoxide, and then 1.0 mL of acetonitrile, 20 µL of nitromethane, and a stirring bar were put therein. Thereafter, 2.08 mL of a 1 M solution of tetra-n-butylammonium fluoride (TBAF) in dimethyl sulfoxide, subjected to a dehydration treatment with molecular sieve 4A, was allowed to flow in at room temperature under stirring with a stirrer, and the mixture was kept at a temperature of 33°C for 4 hours to deprotect the 2'-protecting group. Thereafter, a product of the nucleic acid molecule was obtained by the operation of precipitation. As a result of measuring the purity of the oligonucleotide for the obtained product with the use of the method described in Measurement Method 1, the purity was 67%. In addition, when the yield of oligonucleotide was measured with the use of the method described in Measurement Method 2, the yield was 6068 pg. The results are shown in Table 5.

### (Comparative Example 4)

A nucleic acid molecule was obtained in the same manner as in the experiment according to Example 4, except for the use of the 3% dichloroacetic acid toluene solution prepared in Comparative Example 1 as the 3% dichloroacetic acid toluene solution. As a result of measuring the purity of the oligonucleotide with the use of the method described in Measurement Method 1, the purity of the product was 51%. In addition, when the yield of oligonucleotide was measured with the use of the method described in Measurement Method 2, the yield was 5303 pg. The results are shown in Table 5.

### (Example 5)

A nucleic acid molecule was obtained in the same manner as in the experiment according to Example 4, except for the use of the 3% dichloroacetic acid toluene solution prepared in Example 2 as the 3% dichloroacetic acid toluene solution. As a result of measuring the purity of the oligonucleotide with the use of the method described in Measurement Method 1, the purity of the product was 70%. In addition, when the yield of oligonucleotide was measured with the use of the method described in Measurement Method 2, the yield was 6034 pg. The results are shown in Table 5.

### (Comparative Example 5)

A nucleic acid molecule was obtained in the same manner as in the experiment according to Example 4, except for the use of the 3% dichloroacetic acid toluene solution prepared in Comparative Example 2 as the 3% dichloroacetic acid toluene solution. As a result of measuring the purity of the oligonucleotide with the use of the method described in Measurement Method 1, the purity of the product was 37%. In addition, when the yield of oligonucleotide was measured with the use of the method described in Measurement Method 2, the yield was 4003 pg. The results are shown in Table 5.

### (Example 6)

A nucleic acid molecule was obtained in the same manner as in the experiment according to Example 4, except for the use of the 3% dichloroacetic acid toluene solution prepared in Example 3 as the 3% dichloroacetic acid toluene solution. As a result of measuring the purity of the oligonucleotide with the use of the method described in Measurement Method 1, the purity of the product was 65%. In addition, when the yield of oligonucleotide was measured with the use of the method described in Measurement Method 2, the yield was 6144 pg. The results are shown in Table 5.

### (Comparative Example 6)

A nucleic acid molecule was obtained in the same manner as in the experiment according to Example 4, except for the use of the 3% dichloroacetic acid toluene solution prepared in Comparative Example 3 as the 3% dichloroacetic acid toluene solution. As a result of measuring the purity of the oligonucleotide with the use of the method described in Measurement Method 1, the purity of the product was 43%. In addition, when the yield of oligonucleotide was measured with the use of the method described in Measurement Method 2, the yield was 4492 pg. The results are shown in Table 5.

### [Table 5]

**Table 5**

| | Synthetic sequence | Dichloroacetic acid | | Yield (µg/µmol) | Product purity (%) | Pure yield (yield × product purity) |
|---|---|---|---|---|---|---|
| | | A | B | | | |
| Example 4 | 50 mer | 64 × 10⁻⁷ | 19 × 10⁻⁶ | 6068 | 67% | 4066 |
| Comparative Example 4 | 50 mer | 15 × 10⁻⁴ | 29 × 10⁻⁶ | 5303 | 51% | 2706 |
| Example 5 | 50 mer | 62 × 10⁻⁷ | 21 × 10⁻⁶ | 6034 | 70% | 4224 |
| Comparative Example 5 | 50 mer | 16 × 10⁻⁴ | 26 × 10⁻⁶ | 4003 | 37% | 1481 |
| Example 6 | 50 mer | 56 × 10⁻⁷ | 19 × 10⁻⁶ | 6144 | 65% | 3994 |
| Comparative Example 6 | 50 mer | 11 × 10⁻⁶ | 26 × 10⁻⁵ | 4492 | 43% | 1932 |

| | | | | | | |
|---|---|---|---|---|---|---|
| A: Formaldehyde (mol)/dichloroacetic acid (mol) B: Dichloroacetic anhydride (mol)/dichloroacetic acid (mol) | | | | | | |

From the results in Table 5 above, in Examples 4 to 6 in which the dichloroacetic acid of the present invention was used, nucleic acid molecules were obtained in higher yields and with higher purity as compared with the case of using the dichloroacetic acids in Comparative Examples 4 to 6.

### INDUSTRIAL APPLICABILITY

The present invention provides a production method for purified dichloroacetic acid and an efficient production method for a nucleic acid molecule using the same. In addition, improvement in yield of a nucleic acid molecule produced according to the production method for a nucleic acid molecule can be expected.

### [Sequence Listing Free Text]

The SEQ ID NOs: 1 to 12 of the sequence listing indicate the base sequences of oligonucleotides produced in accordance with the production method of the present invention.

## Claims

1. A production method for purified dichloroacetic acid having a molar ratio of formaldehyde to dichloroacetic acid of 81 × 10⁻⁵ or less and a molar ratio of dichloroacetic anhydride to dichloroacetic acid of 20 × 10⁻⁵ or less, the method comprising:
bringing dichloroacetic acid having both or one of formaldehyde content and dichloroacetic anhydride content exceeding the ratio(s),
into contact with at least one compound having a boiling point lower than that of dichloroacetic acid selected from the group consisting of aliphatic alcohols, aliphatic amines, and water, and, in the coexistence of an aprotic inert solvent having a boiling point lower than that of dichloroacetic acid, distilling off a fraction containing the solvent from the resulting mixed liquid.

2. The production method according to claim 1, wherein the aprotic inert solvent is an aprotic inert solvent having a boiling point of 181°C or lower.

3. The production method according to claim 1 or 2, wherein the aprotic inert solvent is dichloromethane, acetonitrile, or an aromatic organic solvent.

4. The production method according to claim 3, wherein the aromatic organic solvent is toluene.

5. The production method according to any one of claims 1 to 4, wherein the aliphatic alcohol is a C1-C6 aliphatic alcohol.

6. The production method according to any one of claims 1 to 5, wherein the purified dichloroacetic acid has a molar ratio of formaldehyde to dichloroacetic acid of 41 × 10⁻⁵ or less.

7. The production method according to any one of claims 1 to 6, wherein the purified dichloroacetic acid has a molar ratio of dichloroacetic anhydride to dichloroacetic acid of 10 × 10⁻⁵ or less.

8. The production method according to any one of claims 1 to 7, wherein the purified dichloroacetic acid has a molar ratio of formaldehyde to dichloroacetic acid of 81 × 10⁻⁶ or less.

9. The production method according to any one of claims 1 to 8, wherein the purified dichloroacetic acid has a molar ratio of dichloroacetic anhydride to dichloroacetic acid of 50 × 10⁻⁶ or less.

10. A production method for a nucleic acid molecule by an amidite method, the method comprising the steps of: preparing purified dichloroacetic acid having a molar ratio of formaldehyde to dichloroacetic acid of 81 × 10⁻⁵ or less and a molar ratio of dichloroacetic anhydride to dichloroacetic acid of 20 × 10⁻⁵ or less; and reacting the purified dichloroacetic acid with a nucleic acid molecule wherein a hydroxyl group at the 5'-terminal is protected to remove a protecting group for the hydroxyl group.

11. The production method according to claim 10, wherein the preparation step is the method according to any one of claims 1 to 9.

12. The production method according to claim 10 or 11, wherein the nucleic acid molecule wherein a hydroxyl group at the 5'-terminal is protected is a nucleic acid molecule represented by formula (1): 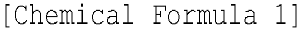 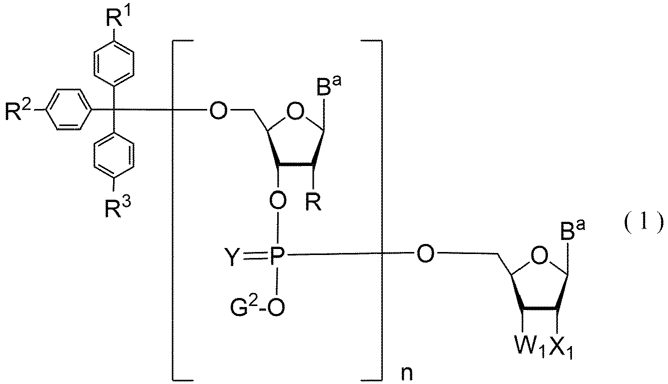 (in the formula,
G² represents a protecting group for a hydroxyl group,
B^{a} is the same or different and each independently represents a nucleobase optionally protected with a protecting group,
R¹, R² and R³ are the same or different and each independently represent a hydrogen atom or an alkoxy group,
R is the same or different and each independently represents a protected hydroxyl group, a hydrogen atom, a fluorine atom, a methoxy group, a 2-methoxyethyl group, or an OQ' group,
Q' is the same or different and each independently represents a methylene group bonded to a carbon atom at a 4'-position of a ribose, an ethylene group bonded to a carbon atom at a 4'-position of a ribose, or an ethylidene group bonded to a carbon atom at a 4'-position of a ribose;
Y is the same or different and each independently represents an oxygen atom or a sulfur atom,
n represents any integer of 1 to 200,
W₁ represents an OZ group, and X₁ represents an R group, or
W₁ represents an OV group, and X₁ represents an OZ group,
V represents a protecting group for a hydroxyl group, and
Z is a group having a structure consisting of a solid support and a linking group, and
when n is an integer of 2 or more, a non-nucleotide linker may be incorporated between respective nucleotides in the nucleic acid molecule represented by the formula (1)), and
the deprotected nucleic acid molecule is a nucleic acid molecule represented by formula (2): 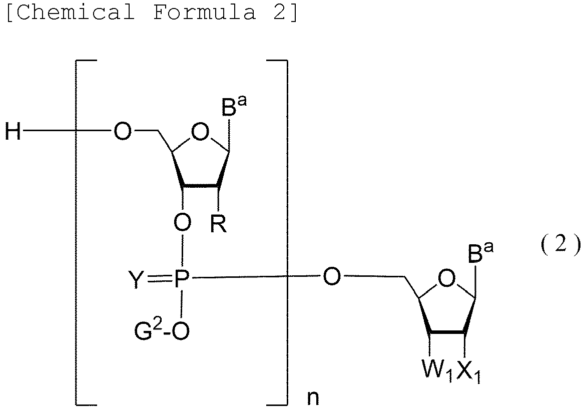 (in the formula,
G², B^{a}, R, Y, X₁, W₁, and n are as described above, and
a non-nucleotide linker may be incorporated between nucleotides as defined in the formula (1)).

13. A production method for a nucleic acid molecule represented by formula (2'), the method comprising: the step according to claim 12; and further a step of removing a group represented by Z from the nucleic acid molecule represented by the formula (2), produced in the step according to claim 12; and a step of removing protecting groups for a hydroxyl group and a nucleobase, 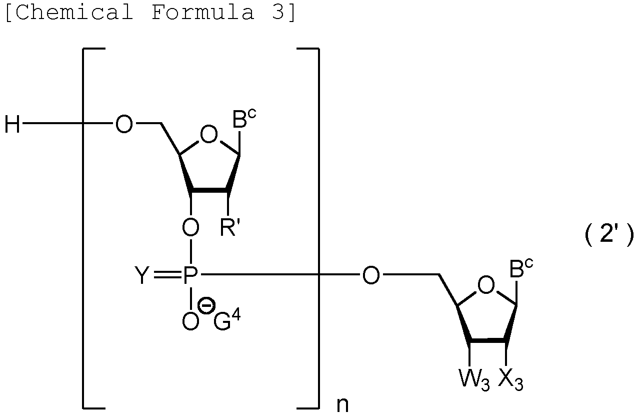 (in the formula,
Y and n are as described above,
B^{c} is the same or different and each independently represents a nucleobase,
G⁴ is the same or different and each independently represents a hydrogen ion, an alkali metal ion, an ammonium ion, an alkylammonium ion, or a hydroxyalkylammonium ion,
R' is the same or different and each independently represents a hydroxyl group, a hydrogen atom, a fluorine atom, a methoxy group, a 2-methoxyethyl group, or an OQ' group,
Q' is as described above, and
X₃ and W₃ each independently represent a hydroxyl group, or
X₃ represents an R' group, and W₃ represents a hydroxyl group, and,
a non-nucleotide linker may be incorporated between nucleotides as defined in the formula (1)).

14. The production method according to any one of claims 10 to 13, wherein the nucleic acid molecule is a nucleic acid molecule containing ribonucleic acid (RNA).

15. The production method according to claim 14, wherein the nucleic acid molecule is ribonucleic acid (RNA), and a protecting group for a hydroxyl group at a 2'-position of a ribose of the ribonucleic acid is a protecting group represented by formula (6),
Formula (6): 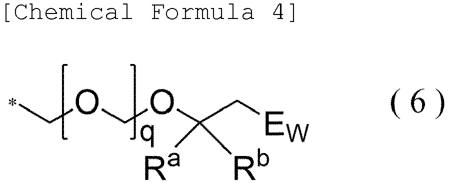 (in the formula,
q represents any integer of 0 to 5,
R^{a} and R^{b} are the same or different and each independently represent a methyl group, an ethyl group, or a hydrogen atom,
mark * represents a site bonded to an oxygen atom derived from the hydroxyl group at the 2'-position of the ribose, and
E_{W} represents an electron-withdrawing group).

16. The production method according to claim 15, wherein one of R^{a} and R^{b} is a methyl group, the other is a hydrogen atom, and E_{w} is a cyano group.

17. The production method according to any one of claims 10 to 16, wherein the nucleic acid molecule is an oligomer with a chain length of 40 or more.

18. The production method according to any one of claims 10 to 16, wherein the nucleic acid molecule is an oligomer with a chain length of 50 or more.

19. The production method according to any one of claims 10 to 16, wherein the nucleic acid molecule is an oligomer with a chain length of 60 or more.

20. The production method according to any one of claims 10 to 16, wherein the nucleic acid molecule is an oligomer with a chain length of 80 or more.

21. The production method according to any one of claims 10 to 16, wherein the nucleic acid molecule is an oligomer with a chain length of 100 or more.

22. An analysis method for dichloroacetic anhydride contained in a dichloroacetic acid reagent, the method comprising: reacting a dichloroacetic acid reagent containing dichloroacetic anhydride with an arylalkylamine to convert the dichloroacetic anhydride to a corresponding 2,2-dichloro-N-arylalkylacetamide; and analyzing the resulting amide by high performance liquid chromatography.

23. The analysis method according to claim 22, wherein
the arylalkylamine is a compound of formula (I): 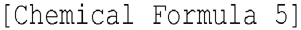 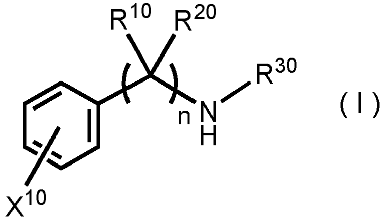 (in the formula,
R¹⁰, R²⁰, and R³⁰ are the same or different and each independently represent a hydrogen atom or an alkyl group,
X¹⁰ represents a hydrogen atom, an alkyl group, or an alkoxy group, and
n is an integer of 1 to 5), and
the 2,2-dichloro-N-arylalkylacetamide is an amide compound of formula (II): 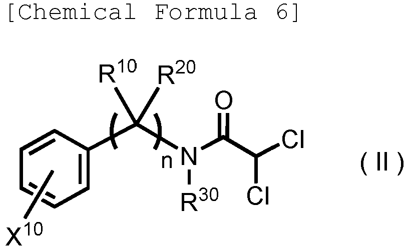 (in the formula, R¹⁰, R²⁰, R³⁰, X¹⁰ and n are as described above).

24. The analysis method according to claim 23, wherein R¹⁰, R²⁰, and R³⁰ are the same or different and each independently represent a hydrogen atom or a C1-C6 alkyl group, and X¹⁰ is a hydrogen atom, a C1-C6 alkyl group, or a C1-C6 alkoxy group.

25. The analysis method according to any one of claims 22 to 24, wherein 0.01 to 3.0 mol of the arylalkylamine is used per 1 mol of dichloroacetic acid.

26. The analysis method according to any one of claims 22 to 24, wherein 0.05 to 2.0 mol of the arylalkylamine is used per 1 mol of dichloroacetic acid.

27. The analysis method according to any one of claims 22 to 24, wherein 0.08 to 1.1 mol of the arylalkylamine is used per 1 mol of dichloroacetic acid.

28. The analysis method according to any one of claims 22 to 27, wherein the arylalkylamine is benzylamine, and the amide compound is 2,2-dichloro-N-benzylacetamide.

29. The analysis method according to any one of claims 22 to 28, wherein the reaction between the dichloroacetic acid reagent containing dichloroacetic anhydride and the arylalkylamine is performed using acetonitrile as a solvent.
